# EUROPEAN PATENT APPLICATION

(11) **EP 0 653 399 A1**
(43) Date of publication of application: **17.05.1995**
(21) Application number: 94203237.6
(22) Date of filing: 07.11.1994
(51) Int. Cl.: C07C 5/27

(54) **Process for the isomerisation of a hydrocarbonaceous feedstock**

(30) Priority: 09.11.1993 EP 93203132
(71) Applicant: SHELL INTERNATIONALE RESEARCH MAATSCHAPPIJ B.V., NL-2596 HR Den Haag (NL)
(72) Inventor: de Jong, Krijn Pieter, NL-1031 CM Amsterdam (NL); Mooiweer, Hendrik Harm, NL-1031 CM Amsterdam (NL); Reinalda, Donald, NL-1031 CM Amsterdam (NL)

(57) **Abstract**

Process for the isomerisation of a hydrocarbonaceous feedstock substantially boiling in the gasoline range which comprises linear paraffins having at least five carbon atoms by contacting the feedstock in the presence of hydrogen at elevated temperature and pressure with a ferrierite catalyst which comprises at least one Group VIII metal component.

## Description

The present invention relates to a process for the isomerisation of a hydrocarbonaceous feedstock substantially boiling in the gasoline range which comprises linear paraffins having at least five carbon atoms.

One of the main objects in nowaday's oil refining is to produce gasolines fulfilling the increasing environmental demands on product quality and having a high octane number.

This means for gasoline that the octane specification has now to be established for instance without lead-containing additives, less aromatics, in particular benzene, and less olefins.

Components which contribute to the octane quality of gasoline include branched paraffins. Object of the present invention is to provide branched paraffins, in particular branched C₇ paraffins.

Therefore, the present invention relates to a process for the isomerisation of a hydrocarbonaceous feedstock substantially boiling in the gasoline range which comprises linear paraffins having at least five carbon atoms by contacting the feedstock in the presence of hydrogen at elevated temperature and pressure with a ferrierite catalyst which comprises at least one Group VIII metal component.

In this way a very attractive high yield of branched paraffins, in particular C₇ paraffins predominantly existing of mono-branched C₇ paraffins, can be obtained. The mono-branched C₇ paraffins obtained in accordance with the present process can as such suitably be applied for the production of multi-branched C₇ paraffins, which constitute even more attractive high octane gasoline components.

Suitably, the ferrierite catalyst to be applied has a SiO₂/Al₂O₃ molar ratio up to 120, preferably from 15 to 100.

Preferably, the ferrierite catalyst to be applied in accordance with the present invention is substantially in its hydrogen form.

It should be noted that in the context of the present invention the term ferrierite catalyst includes apart from ferrierite as such other tectometallosilicates having a ferrierite structure. Such tectometallosilicates include FU-9, ISI-6, Nu-23, ZSM-21, ZSM-35 and ZSM-38. However, ferrierite as such is preferred.

The hydrocarbonaceous feedstock substantially boiling in the gasoline range can suitably be obtained by fractionating any paraffinic gasoline-containing hydrocarbonaceous feedstock. Preferably the feedstock substantially boiling in the gasoline range is obtained by subjecting a straight run hydrocarbon oil to a fractionation, preferably atmospheric distillation. Other suitable hydrocarbonaceous feedstocks substantially boiling in the gasoline range include product fractions obtained from cracking processes such as catalytic cracking, thermal cracking, delayed coking, visbreaking and flexicoking. Hydrocarbonaceous feedstocks containing unacceptable levels of sulphur and nitrogen may be subjected to a hydrotreatment before they are subjected to the process according to the present invention, whereby particularly advantageous results may be obtained. Suitably the hydrocarbonaceous feedstock boiling in the gasoline range has a low sulphur content, preferably less than 10 ppmw on feed. The hydrocarbonaceous feedstock may consist entirely of a fraction boiling in the gasoline range, i.e. in the range of C₄ - 220 °C. While the full gasoline boiling range fraction may be included in the hydrocarbonaceous feedstock, it may be preferred to employ as hydrocarbonaceous feedstock a cut thereof having a boiling point up to 180 °C. Optionally, the hydrocarbonaceous feedstock may be blended with a reformate fraction.

Suitably, the hydrocarbonaceous feedstock substantially boiling in the gasoline range may substantially comprise linear paraffins having at least five carbon atoms, i.e. the feedstock may substantially consist of one or more different types of linear paraffins having at least five carbon atoms. Apart from linear paraffins the feedstock may contain relatively small amounts of branched paraffins having at least five carbon atoms.

Preferably, the hydrocarbonaceous feedstock boiling in the gasoline range substantially comprises linear paraffins having five to ten carbon atoms. More preferably, the hydrocarbonaceous feedstock substantially comprises linear paraffins having six to eight carbon atoms. Suitably, the hydrocarbonaceous feedstock substantially comprises n-heptane. Apart from n-heptane the feedstock may contain C₇ naphthenes and mono-branched C₇ paraffins.

Process conditions which can suitably applied, comprise a temperature of 250 to 350 °C, a pressure of up to 50 bar, a space velocity of 0.5 to 10 g/g/h and a H₂/feedstock molar ratio of less than 5. Preferred conditions comprise a temperature of 275 to 325 °C, a pressure of 10 to 30 bar, a space velocity of 1 to 5 g/g/h and a H₂/feedstock molar ratio of 1 to 3.

Suitably, unconverted linear paraffins are separated from branched hydrocarbons downstream the reaction zone wherein the isomerisation process is performed, and recycled to the reaction zone. Such a separation can, for instance, be carried out by means of a molecular sieve.

The ferrierite catalyst to be used in the process according to the present invention comprises at least one Group VIII metal component. The catalyst may further comprise a binder material comprising one or more refractory oxides. The catalyst may be combined with halogen (e.g. chlorine). The Group VIII metal component may be present on the ferrierite or may be present on a separate carrier (e.g. the binder) comprising, for instance, one or more refractory oxides, e.g. alumina. Preferably, the Group VIII metal component is present on the ferrierite. Suitably, the Group VIII metal component is loaded on the ferrierite by means of ion-exchange or impregnation, although also other methods known in the art may be applied. Suitably, the Group VIII metal component is distributed in the ferrierite, though this may not be achieved by conventional loading techniques. A possible route for incorporation of the Group VIII metal is to add it to the zeolite synthesis mixture. Preferably, a substantial amount of the Group VIII metal component is in metallic form under operating conditions which can be achieved by reduction procedures known in the art. More preferably, the entire amount of the Group VIII metal component is in metallic form. Preferably, the catalyst comprises at least one noble-metal component, more preferably the catalyst comprises a platinum and/or palladium component. Suitably, the catalyst comprises 0.01 to 5% by weight Group VIII metal based on total catalyst. Preferably, the catalyst comprises 0.1 to 3% by weight of Group VIII metal based on total catalyst.

The invention will now be illustrated by means of the following Example.

### Example

An experiment is carried out as follows. A n-heptane feedstock is contacted in the presence of hydrogen with a Pt/ferrierite catalyst (0.25 %wt Pt based on total catalyst, SiO₂/Al₂O₃ molar ratio of 17.6) at a temperature of 301 °C, a pressure of 25 bar, a space velocity of 1.85 kg/(kg.h) and a H₂/Feedstock molar ratio of 2.36. In this way a selectivity towards mono-branched C₇ paraffins yield is obtained of 95.8%, whereas a 53.2% conversion of n-heptane is established.

It will be clear from the above that with the process according to the present invention a very attractive yield of mono-branched C₇ paraffins can be obtained.

## Claims

1. Process for the isomerisation of a hydrocarbonaceous feedstock substantially boiling in the gasoline range which comprises linear paraffins having at least five carbon atoms by contacting the feedstock in the presence of hydrogen at elevated temperature and pressure with a ferrierite catalyst which comprises at least one Group VIII metal component.

2. Process according to claim 1, wherein the ferrierite catalyst has a SiO₂/Al₂O₃ molar ratio of 15 to 100.

3. Process according to claim 1 or 2, wherein the Group VIII metal component comprises a noble-metal component.

4. Process according to any one of claims 1-3, wherein the catalyst comprises 0.01-5% by weight of Group VIII metal based on total catalyst.

5. Process according to any one of claims 1-4, wherein the catalyst comprises a platinum and/or palladium component.

6. Process according to any one of claims 1-5, wherein the catalyst comprises ferrierite.

7. Process according to any one of claims 1-6 wherein the contacting is carried out at a temperature of 250 to 350°C, a pressure of up to 50 bar, a space velocity of 0.5 to 10 g/g/h and a H₂/feedstock molar ratio of less than 5.

8. Process according to any one of claims 1-7, wherein the feedstock substantially comprises linear paraffins having five to ten carbon atoms.

9. Process according to claim 8, wherein the hydrocarbonaceous feedstock substantially comprises linear paraffins having six to eight carbon atoms.
